(19) Europäisches Patentamt
European Patent Office
Office européen des brevets

(11) EP 3 158 008 B2

(12) NOUVEAU FASCICULE DE BREVET EUROPEEN
Après la procédure d'opposition

(45) Date de publication et mention de la décision concernant l'opposition:
**17.05.2023 Bulletin 2023/20**

(45) Mention de la délivrance du brevet:
**25.12.2019 Bulletin 2019/52**

(21) Numéro de dépôt: **15733833.6**

(22) Date de dépôt: **15.06.2015**

(51) Classification Internationale des Brevets (IPC):
***C09D 4/00*** *(2006.01)* ***C08F 222/10*** *(2006.01)*

(52) Classification Coopérative des Brevets (CPC):
**C08F 222/103; C07D 295/13; C08F 222/102;**
C09D 4/00

(86) Numéro de dépôt international:
**PCT/FR2015/051575**

(87) Numéro de publication internationale:
**WO 2015/197941 (30.12.2015 Gazette 2015/52)**

(54) **OLIGOMÈRES ACRYLES MULTIFONCTIONNELS DE STRUCTURE RAMIFIÉE PAR POLYADDITION ENTRE AMINES ET ACRYLATES MULTIFONCTIONNELS**

MULTIFUNKTIONELLE ACRYLOLIGOMERE MIT VERZWEIGTER STRUKTUR DURCH POLYADDITION ZWISCHEN MULTIFUNKTIONELLEN AMINEN UND ACRYLATEN

MULTIFUNCTIONAL ACRYLIC OLIGOMERS OF BRANCHED STRUCTURE, BY POLYADDITION BETWEEN MULTIFUNCTIONAL AMINES AND ACRYLATES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.06.2014 FR 1455789**

(43) Date de publication de la demande:
**26.04.2017 Bulletin 2017/17**

(73) Titulaire: **Arkema France**
**92700 Colombes (FR)**

(72) Inventeurs:
- **CICERON, Philippe**
  **F-60300 Senlis (FR)**
- **BOURROUSSE, Charles**
  **F-75002 Paris (FR)**
- **LEROY, Catherine**
  **F-59000 Lille (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**EP-A1- 1 731 541**
**EP-A1- 1 876 166**
**WO-A1-2014/083644**
**US-A- 5 792 827**
**US-A1- 2002 013 111**

- **anonymous: "Trimethylolpropane triacrylate (TMPTA)", Technical Datasheet, 2019, page 1,**
- **Anonymous: "Glyceryl propoxy triacrylate (OTA 480)", Technical Datasheet, 2019, page 1,**

EP 3 158 008 B2

## Description

La présente invention concerne des oligomères acrylés multifonctionnels de fonctionnalité moyenne supérieure à 2, de structure ramifiée, obtenus par réaction de polyaddition entre une amine multifonctionnelle de fonctionnalité -NH d'au moins 2 et d'un acrylate multifonctionnel de fonctionnalité d'au moins 2, avec une fonctionnalité moyenne sur l'ensemble amine et acrylate supérieure à 2, lesdits oligomères résultant d'une extension de chaîne par ladite polyaddition et par la formation dans l'unité répétitive d'au moins un groupement aminoacrylate dans des conditions spécifiques de rapport molaire acrylate/N-H. De ce fait, lesdits oligomères ont une structure contrôlée, obtenue par un procédé simple en une étape, ayant une réactivité élevée et une viscosité contrôlée pour des applications dans les domaines de revêtements ou des articles en 3D par couches superposées ou le domaine du scellage chimique. L'invention couvre également un procédé spécifique d'obtention desdits produits, une composition réticulable les comprenant, leurs utilisations dans les applications citées et les produits finis obtenus.

Les oligomères selon l'invention ont en particulier comme avantages réunis, à la fois une densité en groupements acrylates par unité de poids et une fonctionnalité en groupement acrylate par mole plus élevées et mieux contrôlées que les produits comparables de l'état de la technique, tout en ayant un taux d'azote et un taux d'aminoacrylate suffisants, en particulier pour un effet synergiste activant la réticulation sous UV et une viscosité faible et convenable pour les applications visées.

L'obtention d'aminoacrylates acrylés en général, monomères ou oligomères, comportant un groupement aminoacrylate est déjà connue de l'état de la technique ainsi que leurs performances pour un effet synergiste par la présence de l'atome d'azote dans le groupement aminoacrylate pour les compositions réticulables sous UV. Ces groupements sont en effet connus pour accélérer ladite réticulation en participant à un mécanisme d'amorçage bimoléculaire, en présence de photoamorceurs tels que la benzophénone, par l'effet donneur électronique de l'atome d'azote qui rend labiles les hydrogènes en $\alpha$ de la fonction amine tertiaire.

US 6,172,129 décrit en particulier des aminoacrylates résultant de l'addition de mono amines secondaires cycliques sur un acrylate multifonctionnel ayant au moins 3 groupements acrylates par molécule. Ces produits ne comportent aucune extension de chaîne possible et ont une structure monomérique, avec en particulier une réduction de la fonctionnalité du produit final par rapport à l'acrylate multifonctionnel de départ et donc ils présentent une plus faible densité de groupements acrylates par unité de poids que ledit acrylate multifonctionnel de départ, malgré leur faible viscosité. Ces produits, pour atteindre un taux élevé de groupements aminoacrylates, contiennent une forte proportion d'espèces saturées résultant de l'addition de monoamine secondaire sur la totalité des groupements acrylates de l'acrylate multifonctionnel de départ.

WO 2011/131501 décrit des amino (méth)acrylates obtenus par réaction d'addition d'une amine avec un mélange d'uréthane (méth)acrylates et d'un diluant réactif (méth)acrylé. Ces produits présentent dans leur structure une extension de chaine par groupement aminoacrylates générés par polyaddition entre une amine primaire ou une diamine secondaire et ledit uréthane (méth)acrylate multifonctionnel. Cependant, ils présentent comme inconvénient d'avoir des viscosités élevées, même en présence de diluant réactif, d'abord par la nature des monomères (méth)acryliques multifonctionnels qui sont des uréthanes et ensuite par l'absence de contrôle stricte de cette extension et de la structure finale. Ceci sans considérer le fait que les groupements méthacrylates, cités comme une possibilité alternative aux acrylates, ne peuvent donner de réaction de polyaddition avec une amine (insuffisance de description avec non faisabilité d'une partie des structures décrites). D'autre part, la densité de groupements acrylates résiduels est plus faible que celle de l'acrylate multifonctionnel de départ avec une viscosité finale plus élevée. US 5 792 827 A divulgue des produits d'addition de Michaël d'oligomères ayant au moins deux fonctions de type ester (meth)acrylique et une polyamine et l'utilisation dans une formulation réticulée sous radiation qui fournit un revêtement caractérisé par sa dureté König.

La présente invention, telle que définie dans les revendications, remédie aux inconvénients cités de l'état de la technique en proposant des nouveaux oligomères acrylés multifonctionnels ayant à la fois une fonctionnalité en groupements acrylates plus élevée que celle de l'acrylate multifonctionnel de départ par l'effet de la présence des groupements acrylates en bouts de chaîne et dans la chaîne principale et par l'effet de la structure ramifiée. Il s'agit en effet d'oligomères comprenant des groupements amino acrylates avec une fonctionnalité moyenne en nombre par oligomère $f_o > 2$ acrylates / mole avec un taux d'azote issu de l'amine $t_A$ élevé ($t_A > 0,35$ mEq/g) tout en ayant une viscosité maîtrisée et restant < 2000 mPa.s à 25°C selon la méthode ISO 2555, sans aucun diluant réactif ajouté. L'avantage d'une viscosité si faible est d'éviter l'addition d'importantes quantités de diluants réactifs. De tels produits ont en particulier une forte réactivité grâce à la combinaison de leur fonctionnalité en acrylate $f_o$ élevée et de l'effet synergiste des fonctions amine, avec amorçages de type I et II possibles en présence de photoamorceur bimoléculaire.

Dans des formulations photoréticulables, le compromis entre vitesse de réticulation, flexibilité, dureté et résistance aux solvants est amélioré par l'utilisation de tels oligomères aminoacrylates acrylés multifonctionnels.

On obtient ces aminoacrylates par réaction de polyaddition (dite addition de Michael ou aza addition) d'amine multifonctionnelle A) de fonctionnalité $f_A$ ($f_A$ : moyenne en nombre si mélange d'amines) d'au moins 2, sur un acrylate B) de fonctionnalité $f_B$ d'au moins 2 ($f_B$ moyenne en nombre si mélange d'acrylates) en excluant le cas où $f_A = f_B = 2$

par le fait que la fonctionnalité moyenne en nombre sur l'ensemble des composants A) et B) doit être supérieure à 2, permettant ainsi une structure ramifiée maitrisée. Structure « ramifiée » également dite « branchée » signifie que la chaîne d'oligomère obtenue comporte au moins une ramification ou branchement de chaîne avec la même unité répétitive. La maîtrise de l'accroissement de masse moléculaire de ces systèmes et donc de leur viscosité est assurée par le rapport r du nombre de doubles liaisons acrylates par rapport au nombre de fonctions amines -NH (r = Acrylate / NH), la conversion x étant supérieure à 95% par rapport aux fonctions -NH réactives qui sont en défaut par rapport aux fonctions acrylates B). La viscosité de tels systèmes reste maîtrisable sans conduire à la gélification du milieu réactionnel par la limitation spécifique dudit rapport r qui reste supérieur à une valeur spécifiquement définie et dépendante des fonctionnalités des deux composants pour une conversion donnée. Dans tous les cas, le rapport r = acrylate/NH reste supérieur à 1 pour que l'oligomère soit acrylé comme défini selon l'invention.

Parmi les principaux avantages de la présente invention par rapport à l'état de la technique, on peut citer les suivants :

Pour le procédé d'obtention :

- Simple réaction d'addition (Aza ou Michael) de l'amine multifonctionnelle A) sur l'acrylate B) avec un mélange réactionnel et en une seule étape de procédé.
- Pas d'effluent gazeux ou de sous-produits à éliminer avec un rendement de 100% par rapport au poids des réactants.
- Pas besoin de solvant ni de catalyseur, avec température de réaction d'environ 80°C et temps de réaction court avec un moindre impact sur l'environnement.
- Reproductibilité et prédictibilité des structures et des propriétés finales sur la base des critères de l'invention.

Pour le produit :

- Viscosité maîtrisée avec une valeur < 2000 mPa.s à 25°C selon ISO 2555.
- Taux d'azote $t_A$ élevé, issu de l'amine multifonctionnelle A) avec $t_A$ supérieur ou égal à 0,35 mEq/g.
- Bon compromis entre fonctionnalité acrylate moyenne en nombre par oligomère, $f_o$ et la densité de groupements acrylates par unité de poids $t_{acr}$, avec respectivement $f_o > 2$ et $t_{acr} > 2,3$ mEq/g.
- Faible taux de migrables avec absence d'espèces saturées complètement (absence de saturation complète des acrylates étant donné l'excès significatif des fonctions acrylates par rapport aux fonctions amines).
- Maîtrise de l'allongement de chaîne par l'ajustement du rapport r = Acrylate / NH et contrôle de la structure sans risque de gélification, malgré la ramification de la chaîne.

Par rapport aux applications visées dans des compositions réticulables par rayonnement et en particulier UV ou par voie peroxyde comprenant lesdits oligomères de l'invention, on a comme avantages une réactivité élevée desdits oligomères avec une flexibilité, dureté et résistance aux solvants élevées des produits réticulés obtenus.

L'invention couvre d'abord l'oligomère acrylé de structure ramifiée, comme défini selon l'invention.

Ensuite, elle concerne un procédé spécifique de préparation dudit oligomère.

Un autre objet concerne une composition réticulable comprenant ledit oligomère.

Fait également partie de l'invention, l'utilisation dudit oligomère dans les compositions réticulables.

Finalement, l'invention concerne les produits finis réticulés obtenus en utilisant ledit oligomère ou une composition réticulable le contenant.

Donc, le premier objet de l'invention concerne un oligomère acrylé, lequel a une fonctionnalité en acrylate moyenne en nombre (ou fonctionnalité moyenne en nombre en acrylate) $f_o$ strictement supérieure à 2 acrylates par mole, lequel oligomère a une structure ramifiée et résulte de la réaction de polyaddition d'au moins une amine multifonctionnelle A) de fonctionnalité $f_A$ en groupements N-H d'au moins 2, de préférence de 2 à 6, avec ladite fonctionnalité en N-H signifiant une fonctionnalité moyenne en nombre s'il s'agit d'un mélange d'amines, ladite amine multifonctionnelle A) étant porteuse de fonctions amines primaires et/ou secondaires avec en option ladite amine multifonctionnelle A) étant porteuse en plus d'au moins une fonction amine tertiaire (c'est-à-dire sans N-H), avec ladite polyaddition sur au moins un acrylate multifonctionnel B) de fonctionnalité $f_B$ en groupements acrylates d'au moins 2, de préférence de 2 à 6, avec ladite fonctionnalité $f_B$ en acrylates signifiant une fonctionnalité moyenne en nombre s'il s'agit d'un mélange d'acrylates, avec une fonctionnalité moyenne en nombre par mole sur l'ensemble des composants A et B supérieure à 2 et avec ledit oligomère comprenant dans sa structure d'unité répétitive au moins un groupement aminoacrylate $-O_2C-CH_2-CH_2-N=$ résultant de ladite polyaddition et avec un taux d'azote $t_A$ issu de ladite amine multifonctionnelle A) supérieur ou égal à 0,35 mEq/g et un rapport initial r = acrylate / N-H compris entre $r_{inf}$ et 1,1 $r_{sup}$ et de préférence r compris entre $r_{inf}$ et $r_{sup}$, avec les valeurs $r_{inf}$ et $r_{sup}$ étant définies selon les relations (1) et (2) suivantes :

$$r_{inf} = 0,90*(f_A-1)*(f_B -1) \quad (1)$$

$$r_{sup} = 2*f_A + 2*f_B - 6 \quad (2)$$

et avec un nombre moyen $n_{moy}$ de motifs répétitifs (ou unités répétitives) par oligomère (en plus du monomère B) défini selon la relation (3) suivante :

$$n_{moy} = 1 / [(r*f_A/f_B) + 1 - f_A] \quad (3)$$

Ledit motif répétitif correspond à une unité aminoacrylate, formée par la réaction d'addition d'un groupement NH de ladite amine multifonctionnelle A) sur un groupement acrylate dudit acrylate multifonctionnel B).
Selon une préférence particulière, la somme $f_A + f_B$ reste inférieure ou égale à 8.
Plus particulièrement, $n_{moy}$ peut varier de 0,1 à 5, de préférence de 0,15 à 3.
Selon une première option préférée, ladite amine multifonctionnelle A) porte en plus, au moins une fonction amine tertiaire, de
préférence ladite amine multifonctionnelle étant choisie parmi diméthylamino propylamine (DMAPA), diméthylamino propylamino propy-lamine (DMAPAPA) et 1,4 Bis (3-aminopropyl) pipérazine (1,4-BAPP) et plus préférentiellement parmi DMAPAPA et 1,4-BAPP et avec un taux d'azote $t_A$ supérieur ou égal à 0,35 mEq/g. Le taux d'azote, dans ce cas, englobe l'azote de toutes les fonctions amine, y compris celui des fonctions amine tertiaire présentes dans ce cas préféré.
La viscosité dudit acrylate B) mesurée selon la méthode ISO 2555 à 23°C est de préférence inférieure à 200 mPa.s sous un cisaillement de 100 $s^{-1}$. Ledit acrylate B) est plus particulièrement sélectionné parmi : b1) acrylates de polyols aliphatiques ou cycloaliphatiques éventuellement alcoxylés, b2) oligoéthers acrylates, b3) acrylates phénoliques alcoxylés ou b4) aminoacrylates acrylés ou leurs mélanges et de préférence selon b1) ou b4) ou leurs mélanges. Un mélange d'acrylates B) peut donc être un mélange d'acrylates B) de même type, c'est-à-dire par exemple un mélange entre au moins deux acrylates de type b1) ou d'au moins deux acrylates de type b2) ou d'au moins deux acrylates de type b3) ou d'au moins deux acrylates de type b4), en particulier d'au moins deux acrylates de type b1) ou b2) et leurs mélanges entre types différents.
Ladite amine multifonctionnelle A) est de préférence choisie parmi : a1) une amine aliphatique, a2) une amine cycloaliphatique ou a3) une amine aralkylène avec la fonction amine en position autre que la position en alpha (alpha signifiant que N est lié directement au noyau) ou autre que la position en beta (avec N porté par un carbone en alpha du noyau aromatique) du noyau aromatique ou leurs mélanges, de préférence a1)aliphatique et/ou a2) cycloaliphatique y compris polycycliques ou leurs mélanges. Plus précisément, dans le cas particulier a3), la fonction amine n'est, ni en position alpha, ni en position beta, par rapport au noyau aromatique.
Ladite amine multifonctionnelle A) peut être représentée par une formule générale $(NH_2)_{fA1}R2(NHR3)_{fA2}$, avec R2 = R'(NR"R"')$_{fA3}$, R3, R', R" et R'" étant des alkyles en $C_1$ à $C_3$ identiques ou différents avec $f_{A1}$ étant le nombre de fonctions amines primaires par mole, $f_{A2}$ étant le nombre de fonctions amines secondaires par mole et $f_{A3}$ étant le nombre de fonctions amines tertiaires par mole et avec $f_A = 2*f_{A1}+f_{A2}$.
Ledit acrylate B) peut être représenté par la formule générale $R1(X)_{fB}$ avec X étant un groupement acrylate $CH_2$=CH-$CO_2$- et R1 étant le résidu dudit acrylate B) porteur de $f_B$ acrylates par mole.
L'oligomère acrylé selon l'invention a en particulier un taux de groupements acrylates ou une densité en groupements acrylates $t_{acr}$ supérieure à 2,3 et de préférence de 3,5 à 10 mmole/g ou milli équivalent/g (mEq/g).
Ledit oligomère peut être caractérisé par une masse moléculaire moyenne en nombre calculée Mn allant de 275 à 5000, de préférence de 300 à 3000, avec Mn étant définie selon la relation (4) suivante :

$$Mn = M_B + (n_{moy} * M_u) \quad (4)$$

avec $M_u$ étant la masse molaire de l'unité répétitive définie selon la relation (5) suivante :

$$M_u = M_A + (f_{A2} + 2*f_{A1} - 1)*M_B \qquad (5)$$

$n_{moy}$ étant le nombre moyen d'unités répétitives tel que défini dans la revendication 1, $M_B$ étant la masse molaire de l'acrylate B),
$M_A$ étant la masse molaire de l'amine multifonctionnelle A),
$f_{A1}$ étant le nombre de fonctions $-NH_2$ d'amine primaire, par amine multifonctionnelle A) et
$f_{A2}$ étant le nombre de fonctions -NH- d'amine secondaire, par amine multifonctionnelle A).

Concernant le taux d'azote $t_A$ dans ledit oligomère de l'invention, il peut varier de 0,4 à 5, de préférence de 0,45 à 4 mEq/g, avec $t_A$ étant défini selon la relation (6) suivante :

$$t_A = 1000*n_{moy}*(f_{A1} + f_{A2} + f_{A3}) / Mn \qquad (6)$$

avec Mn étant la masse moléculaire moyenne en nombre telle que définie ci-haut et
$f_{A1}$ étant le nombre de fonctions $-NH_2$ amine primaire, par amine multifonctionnelle A) et
$f_{A2}$ étant le nombre de fonctions -NH- amine secondaire, par amine multifonctionnelle A) et
$f_{A3}$ étant le nombre de fonctions -N= amine tertiaire, par amine multifonctionnelle A).

La fonctionnalité $f_o$, exprimée en équivalent de doubles liaisons (acrylates) par mole dudit oligomère peut être définie selon la relation (7) suivante :

$$f_o = f_B + n_{moy}*(f_{A2}*f_B - f_{A2} - f_B + 2*f_{A1}*f_B - 2*f_{A1}) \qquad (7)$$

$f_o$ ainsi définie peut varier de 2,1 à 6, de préférence de 2,3 à 5.

Le taux d'acrylate $t_{acr}$, exprimé en milliéquivalents (ou mmoles) de double liaison par gramme dudit oligomère, peut être défini selon la relation (8) suivante :

$$t_{acr} = 1000 * f_o / M_n \qquad (8)$$

Ledit acrylate B) peut être alcoxylé et dans ce cas de préférence le nombre de motifs alcoxy par groupement acrylate ne dépasse pas 3 si ledit alcoxy est l'éthoxy et ne dépasse pas 1 si ledit alcoxy est le propoxy.

Plus particulièrement, concernant les deux réactants A) et B), ladite amine multifonctionnelle A) a une fonctionnalité $f_A$ supérieure ou égale à 2 et ledit acrylate B) a une fonctionnalité $f_B$ supérieure ou égale à 3 ou inversement ladite amine multifonctionnelle A) a une fonctionnalité $f_A$ supérieure ou égale à 3 et que ledit acrylate B) a une fonctionnalité $f_B$ supérieure ou égale à 2 et plus particulièrement $f_A + f_B$ ne dépasse pas 8 et de préférence inférieure à 8.

Selon une option particulière où il y a absence de fonctions $-NH_2$ amine primaire dans ladite amine multifonctionnelle A) ($f_{A1} = 0$ dans la formule générale de l'amine A)), ledit oligomère peut être défini par le fait que

- ladite amine multifonctionnelle A) est de formule générale $(NH_2)_{fA1}R2(NHR3)_{fA2}$, avec $R2 = R'(NR''R''')_{fA3}$, R3, R', R'' et R''' étant des alkyles en $C_1$ à $C_3$ identiques ou différents avec $f_{A1}$ étant égal à 0 et étant le nombre de fonctions amines primaires par mole, $f_{A2}$ étant supérieur ou égal à 2 et étant le nombre de fonctions amines secondaires par mole et $f_{A3}$ étant supérieur ou égal à 0 et étant le nombre de fonctions amines tertiaires par mole
- et avec $f_A = f_{A2}$ ledit acrylate B) est de formule générale $R1(X)_{fB}$ avec X étant un groupement acrylate $CH_2=CH-CO_2-$ et R1 étant
- le résidu dudit acrylate B) porteur de $f_B$ acrylates par mole et que
  ledit oligomère comprend le produit de formule générale (I) suivante :

$$X_{(fB-1)}R1[O_2CCH_2CH_2NR3R2Y_{(fA2-2)}NR3CH_2CH_2CO_2R1X_{(fB-2)}]_nX \qquad (I)$$

avec $Y = -(NR3CH_2CH_2COOR1X_{(fB-1)})$

La formule plus développée et détaillée de la formule (I) peut correspondre à la formule suivante avec tous les paramètres utilisés étant définis comme ci-haut pour ladite formule (I) :

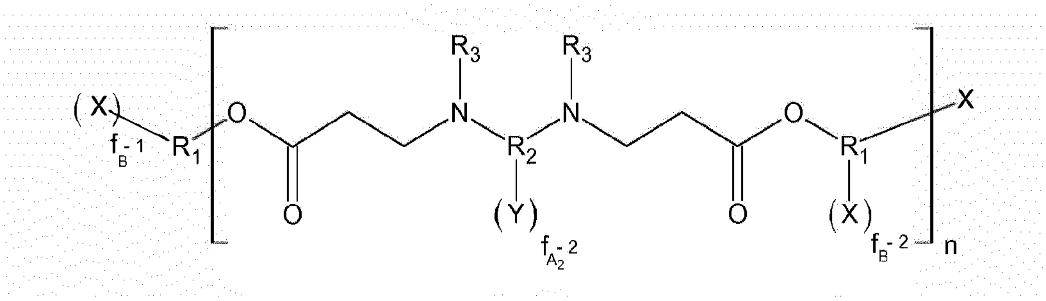

avec :

Y :

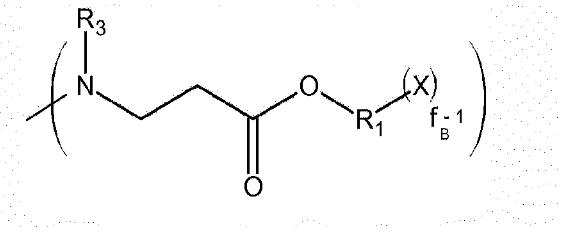

Selon une autre option particulière avec présence de fonction amine primaire et secondaire, ledit oligomère peut être défini par le fait que :

- ladite amine <u>multifonctionnelle</u> A) est de formule générale $(NH_2)_{fA1}R2(NHR3)_{fA2}$, avec R2 = $R'(NR''R''')_{fA3}$, R3, R', R" et R'" étant des alkyles en $C_1$ à $C_3$ identiques ou différents, $f_{A1}$ étant supérieur ou égal à 1 et étant le nombre de fonctions amines primaires par mole, $f_{A2}$ étant supérieur ou égal à 0 et étant le nombre de fonctions amines secondaires par mole et $f_{A3}$ étant supérieur ou égal à 0 et étant le nombre de fonctions amines tertiaires par mole et avec

$$f_A = 2*f_{A1} + f_{A2}$$

- ledit acrylate B) est de formule générale $R1(X)_{fB}$ avec X étant un groupement acrylate $CH_2$=CH-$CO_2$- et R1 étant le résidu dudit acrylate B) porteur de $f_B$ acrylates par mole et que
- ledit oligomère comprend le produit de formule générale (II) suivante :

$$X_{(fB-1)}R1[O_2CCH_2CH_2NR2Y_{fA2}Z_{(fA1-1)}CH_2CH_2CO_2R1X_{(fB-2)}]_nX \qquad (II)$$

avec Y = $-(NR3CH_2CH_2CO_2R1X_{(fB-1)})$ et
Z = $-(N(CH_2CH_2COOR1X_{(fB-1)})_2)$.

La formule plus développée et détaillée de la formule (II) peut correspondre à la formule suivante avec tous les paramètres utilisés étant définis comme ci-haut pour ladite formule (II) :

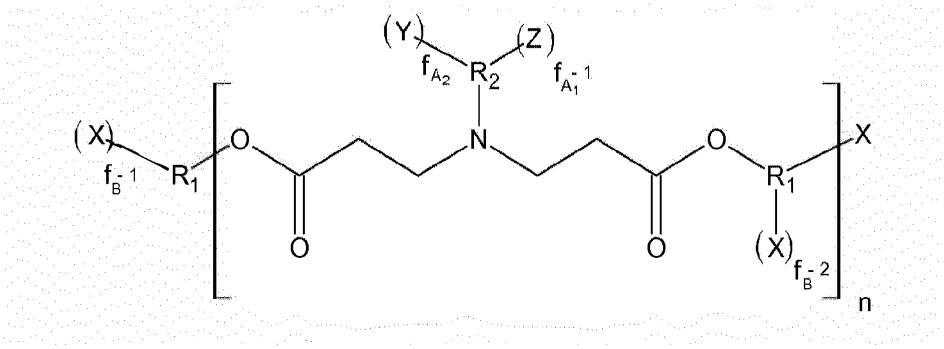

avec

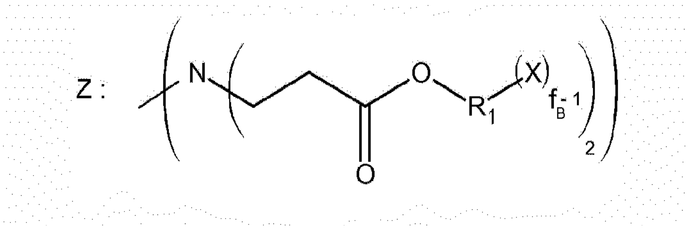

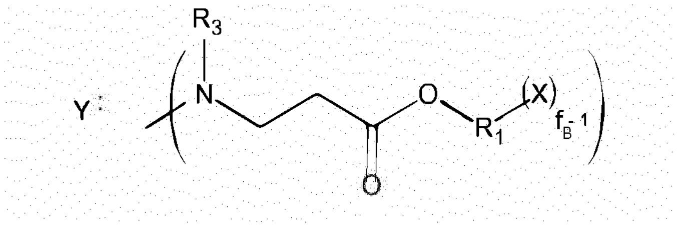

Selon une autre possibilité, ladite fonctionnalité $f_A$ de l'amine multifonctionnelle A) est égale à 3 et ladite amine multifonctionnelle A) est sélectionnée parmi une diamine porteuse d'une fonction amine primaire et d'une fonction amine secondaire ou parmi une triamine porteuse de 3 fonctions amines secondaires.

Selon une autre variante, ladite amine multifonctionnelle A) a une fonctionnalité $f_A$ égale à 2 et est une amine primaire, c'est-à-dire porteuse d'une fonction amine primaire $-NH_2$ ou est une diamine porteuse de 2 fonctions amine secondaire et ledit acrylate B) est un triacrylate éventuellement alcoxylé avec, dans le cas où ledit triacrylate est alcoxylé, un nombre de motifs alcoxy par acrylate ne dépassant pas 3 si ledit alcoxy est l'éthoxy et un nombre de motifs alcoxy par acrylate ne dépassant pas 1 si ledit alcoxy est propoxy.

Selon une autre variante possible, ladite amine multifonctionnelle A) a une fonctionnalité $f_A$ égale à 3 et est une diamine primaire-secondaire, c'est-à-dire porteuse d'une fonction amine primaire et d'une fonction amine secondaire ou est une triamine porteuse de 3 fonctions amines secondaires et ledit acrylate B) est un diacrylate éventuellement alcoxylé, avec dans le cas où ledit diacrylate est alcoxylé, un nombre de motifs alcoxy par acrylate ne dépassant pas 2 si ledit alcoxy est l'éthoxy et un nombre de motifs alcoxy par acrylate ne dépassant pas 1 si ledit alcoxy est le propoxy.

Selon une autre option, ladite fonctionnalité $f_A$ de l'amine multifonctionnelle A) est égale à 4 et ladite amine A) est sélectionnée parmi une diamine porteuse de 2 fonctions amines primaires ou une triamine porteuse de 1 fonction amine primaire et de 2 fonctions amines secondaires ou une tetra-amine porteuse de 4 fonctions amines secondaires.

Encore selon une option différente, ladite fonctionnalité $f_A$ est égale à 5 et ladite amine multifonctionnelle A) est sélectionnée parmi une triamine porteuse de deux fonctions amines primaires et d'une fonction amine secondaire ou une tetra-amine porteuse d'une fonction amine primaire et de trois fonctions amines secondaires ou une penta-amine porteuse de 5 fonctions amines secondaires.

Encore selon une autre option, ladite fonctionnalité $f_A$ est égale à 6 et ladite amine multifonctionnelle A) est sélectionnée parmi une triamine porteuse de trois fonctions amines primaires ou une tetra-amine porteuse de 2 fonctions amines primaires et de 2 fonctions amines secondaires ou une penta-amine porteuse de 1 fonction amine primaire et de 4 fonctions amines secondaires ou une hexa-amine porteuse de 6 fonctions amines secondaires.

Ladite amine multifonctionnelle A) comme déjà mentionné ci-haut peut être un mélange d'amines primaires

et/ou secondaires comme définies ci-haut. Dans ce cas, la fonctionnalité $f_A$ utilisée est la fonctionnalité moyenne en nombre sur l'ensemble des amines utilisées dans ledit mélange. Il est possible selon une option particulière qu'une amine secondaire mono-fonctionnelle, en particulier cycloaliphatique, soit présente dans ledit mélange, à partir du moment où les proportions des amines composants du mélange et leur fonctionnalités en groupements NH sont telles que la fonctionnalité moyenne en nombre $f_A$ en groupements NH dudit mélange d'amines, est d'au moins 2 et de préférence de 2 à 6.

L'oligomère de l'invention, de structure ramifiée, a une fonctionnalité moyenne en nombre en acrylates fo allant de 2,1 à 6, de préférence de 2,3 à 5 par oligomère, c'est-à-dire par mole d'oligomère.

Selon une option particulière, ledit oligomère peut résulter de la réaction entre au moins deux amines A) et/ou d'au moins deux acrylates B). Cela signifie qu'on peut faire réagir selon l'invention au moins deux amines A) avec au moins un acrylate B) ou une amine multifonctionnelle A) avec au moins deux acrylates B) ou au moins deux amines A) avec au moins deux acrylates B).

Ledit oligomère selon l'invention a de préférence une masse moléculaire moyenne en nombre Mn calculée selon la relation (4) définie ci-dessus, allant de 275 à 5000 (en g/mole ou Dalton) et de préférence de 300 à 3000.

L'oligomère, selon l'invention, comprend n unités répétitives avec une distribution moléculaire en fonction de n (nombre d'unités répétitives), ce qui signifie qu'il présente une distribution moléculaire en fonction de n et en particulier il comprend au moins les 4 produits correspondant à : n = 0 et n = 1 et n = 2 et n = 3 et plus particulièrement avec au moins 50% en poids de ladite distribution correspondant à n inférieur ou égal à 3. Plus particulièrement, ladite distribution moléculaire en fonction de n comprend au moins les 5 produits correspondant à : n = 0 et n = 1 et n = 2 et n = 3 et n = 4 et encore plus particulièrement avec au moins 60% en poids de ladite distribution correspondant à n inférieur ou égal à 4.

Le deuxième objet de l'invention concerne un procédé de préparation dudit oligomère tel que défini selon l'invention ci-dessus, lequel procédé comprend une étape de réaction de polyaddition (addition dite de Michael) de ladite au moins une amine multifonctionnelle A) sur ledit au moins un acrylate B), en présence d'un inhibiteur de polymérisation radicalaire et en l'absence de tout solvant, de tout catalyseur et de tout autre réactant hormis ladite amine multifonctionnelle A) et ledit acrylate B) et inhibiteur, ledit procédé comprenant l'addition progressive et continue de ladite amine multifonctionnelle A) sur ledit acrylate B) déjà présent dans le réacteur et B) toujours en excès stœchiométrique et avec ladite réaction ayant lieu à une température supérieure à 40°C et inférieure à 90°C, de préférence de 60 à 80°C et avec ladite réaction étant arrêtée à un taux de conversion des fonctions amines N-H d'au moins 95% et avec un rapport initial r de fonctions acrylates sur lesdites fonctions amines N-H, r = acrylate / N-H étant entre $r_{inf}$ et 1,1 $r_{sup}$ et de préférence r compris entre $r_{inf}$ et $r_{sup}$, avec les valeurs $r_{inf}$ et $r_{sup}$ étant définies selon les relations (1) et (2) suivantes

$$r_{inf} = 0{,}90*(f_A-1)*(f_B-1) \qquad (1)$$

$$r_{sup} = 2*f_A + 2*f_B - 6 \qquad (2)$$

Un autre objet de l'invention concerne une composition réticulable, laquelle comprend au moins un oligomère tel que défini ci-haut ou obtenu par le procédé tel que défini selon l'invention, avec en option présence d'un diluant réactif, en particulier choisi parmi les monomères (méth)acryliques mono et/ou multifonctionnels et avec ledit diluant réactif pouvant être identique audit acrylate de départ B) ou ledit diluant pouvant être différent. Ledit diluant réactif peut en particulier être utilisé pour des valeurs des viscosités les plus élevées de la plage < 2000 mPa.s. Ledit diluant identique à B) est par définition multifonctionnel acrylique, comme B). Si différent de B), il peut être différent par la nature de la fonctionnalité (méth)acrylique, c'est-à-dire porter des groupements méthacrylates ou être monofonctionnel acrylate ou méthacrylate ou multifonctionnel (acrylate ou méthacrylate) de nature différente ou de fonctionnalité (groupements acrylates ou méthacrylates par mole) différente de celle de B) (en acrylates). Ledit diluant réactif en dehors de son rôle de diluant réactif pour ajuster la viscosité à celle visée pour l'application finale peut également servir pour ajuster les performances finales du produit réticulé obtenu à partir de ladite composition réticulable.

Plus particulièrement, ladite composition (réticulable) est une composition réticulable par rayonnement de préférence parmi UV, laser, LED ou EB, qui est une composition de revêtements, en particulier d'encres, de vernis, d'enduits gélifiés (couramment appelés « gel coats »), de peintures ou d'adhésifs, en particulier d'adhésifs structuraux ou est une composition pour articles en 3D par superposition de couches successives ou est une composition de moulage.

Selon une autre variante, ladite composition est une composition réticulable par voie peroxyde (appelée couramment « P-cure ») et en particulier c'est une composition de revêtements plus particulièrement de vernis, d'enduits gélifiés (« gel coats »), de peintures ou d'adhésifs, en particulier d'adhésifs structuraux ou est une composition de

scellage chimique ou une composition de moulage.

Les compositions de moulage sont des compositions réticulables pour pièces moulées et en particulier pour matériaux de structure, y compris pour matériaux composites renforcés par des renforts fibreux.

Un autre objet de l'invention concerne l'utilisation d'un oligomère tel que défini ci-haut comme liant dans une composition réticulable. Selon une première option de ladite utilisation, ladite composition est réticulable par rayonnement de préférence parmi UV, laser, LED ou EB, ladite composition étant une composition de revêtements, en particulier d'encres, de vernis, d'enduits gélifiés (couramment appelés « gel coats »), de peintures ou d'adhésifs, en particulier d'adhésifs structuraux ou étant une composition pour articles en 3D par superposition de couches successives ou étant une composition de moulage.

Selon une autre variante de ladite utilisation, il s'agit d'utilisation dans une composition réticulable par voie peroxyde (appelée couramment « P-cure ») qui est en particulier une composition de revêtements, plus particulièrement de vernis, d'enduits gélifiés (« gel coats »), de peintures ou d'adhésifs, en particulier d'adhésifs structuraux ou une composition de scellage chimique ou une composition de moulage.

Finalement, l'invention concerne le produit final réticulé qui résulte de l'utilisation d'au moins un oligomère tel que défini ci-haut ou obtenu par le procédé défini ci-haut ou qui résulte de la réticulation d'une composition telle que définie ci-haut selon l'invention et en particulier qui est un film de revêtement, plus particulièrement un film d'encre, de vernis, de revêtement d'enduits gélifiés, d'adhésif ou qui est un article en 3D ou un joint de scellage chimique ou une pièce moulée.

Les exemples suivants sont donnés à titre d'illustration de l'invention et de ses performances et ne limitent d'aucune manière sa portée.

Partie expérimentale

Matières premières : voir tableau 1 ci-dessous

Tableau 1 : matières premières

| Nom commercial (REF) | Nom chimique | Nom abrégé | Fournisseur | Fonction selon l'invention | Fonctionnalité |
|---|---|---|---|---|---|
| SR341 | 3 Méthyl 1,5 Pentane Diol DiAcrylate | 3M15PDDA | SARTOMER | Acrylate B) | $f_B = 2$ |
| SR238 | HexaneDiol DiAcrylate | HDDA | SARTOMER | Acrylate B) | $f_B = 2$ |
| SR455LM | TriMéthylol Propane TétraEthoxylé TriAcrylate | TMP4EOTA | SARTOMER | Acrylate B) | $f_B = 3$ |
| SR454 | TriMéthylol Propane TriEthoxylé TriAcrylate | TMP3EOTA | SARTOMER | Acrylate B) | $f_B = 3$ |
| SR351 | TriMéthylol Propane TriAcrylate | TMPTA | SARTOMER | Acrylate B) | $f_B = 3$ |
| DMAPA | 3-(DiMéthylAmino)1-Propyle Amine | DMAPA | ALDRICH | Amine multifonctionnelle A) | $f_A = 2$ |
| DMAPAPA | Diméthyl Amino PropylAmino PropylAmine | DMAPAPA | ARKEMA | Amine multifonctionnelle A) | $f_A = 3$ |
| 1,3BAC | 1,3Bis (AminométhylCyclo hexane) | 1,3 BAC | MITSUBISHI | Amine multifonctionnelle A) | $f_A = 4$ |

(suite)

| Nom commercial (REF) | Nom chimique | Nom abrégé | Fournisseur | Fonction selon l'invention | Fonctionnalité |
|---|---|---|---|---|---|
| 1,4BAPP | 1,4Bis(3-AminoPropyl) Pipérazine | 1,4 BAPP | ALDRICH | Amine multifonctionnelle A) | $f_A = 4$ |
| EMHQ | Ether Méthylique d'hydroquinone | EMHQ | RHODIA | Inhibiteur | |
| Darocure® 1173 | 2 Hydroxy 2 Méthyl1 Phényle Propane1-one | Dar1173 | CIBA | Photoamorceur | |
| Benzophénone | Diphényl Méthanone | BzPh | ARKEMA | Photoamorceur | |

Exemples de préparation d'oligomères selon l'invention

Exemple A1

Dans un réacteur de 1 litre équipé d'un agitateur à ancre et surmonté d'un simple réfrigérant ascendant, dispositif permettant la mise à l'atmosphère du milieu réactionnel, d'une entrée d'air (barbotage d'air), d'une ampoule de coulée et d'une sonde thermométrique, on introduit : 178,4 g d'hexanediol diacrylate (HDDA) (0,7895 mole) et 84,7 mg d'éther méthylique d'hydroquinone (EMHQ).

Puis, on introduit progressivement dans le réacteur par l'ampoule de coulée en une heure : 33,5 g de diméthylamino propylamino propylamine (DMAPAPA) (0,2105 mole) pendant que le mélange réactionnel est porté progressivement à 80°C et maintenu à cette température jusqu'à une conversion supérieure à 95% des fonctions amine réactives primaires et secondaires ($1^{aire}$ + $2^{aire}$) et stabilisation de la viscosité, soit 10 heures de réaction. Après refroidissement à température ambiante, on obtient un produit ayant un aspect limpide, jaune clair.

Exemple A2

Réalisé à l'identique par rapport à l'exemple A1, sauf en remplaçant à poids égal pour A) l'HDDA par 178,4 g de 3-méthyl 1,5-pentane diol diacrylate (3M1,5PDDA) (0,7895 mole). On obtient un produit ayant un aspect limpide, jaune clair.

Exemple A3

Réalisé à l'identique par rapport à l'exemple A1, sauf qu'on modifie les réactifs acrylate B) et amine A), comme suit : pour B), 188,49 g de dipropylèneglycol diacrylate (DPGDA) (0,7895 mole) et pour A), 35,15 g de diméthylamino propylamino propylamine (DMAPAPA) (0,2105 mole). On obtient un produit ayant un aspect limpide, jaune clair.

Les compositions molaires et caractéristiques des 3 oligomères obtenus A1, A2 et A3 sont présentés ci-dessous dans le tableau 2.

Tableau 2 : Composition molaire et caractéristiques des oligomères A1, A2 et A3

| Référence d'oligomère par exemple | A1 | A2 | A3 |
|---|---|---|---|
| Composant réactif (moles) | | | |
| A) : 3M1,5PDDA | | 0,7895 | |
| B) : DPGDA | | | 0,7895 |
| B) : HDDA | 0,7895 | | |
| A) : DMAPAPA | 0,2105 | 0,2105 | 0,2105 |
| Caractéristiques | | | |
| r | 2,50 | 2,50 | 2,35 |

(suite)

| Caractéristiques | | | |
|---|---|---|---|
| $t_A$ (mEq/g) | 2,94 | 2,94 | 2,69 |
| Viscosité à 25°C (mPa.s) | 255 | 250 | 590 |
| $t_{acr}$ (mEq/g) | 4,47 | 4,47 | 4,08 |
| $f_o$ (mEq/mole) | 2,57 | 2,57 | 2,57 |
| $n_{moy}$ | 0,57 | 0,57 | 0,57 |
| $M_{n\ calc}$ | 575 | 575 | 631 |

Exemple B1

Réalisé à l'identique par rapport à l'exemple A1, sauf qu'on modifie les réactifs acrylate B) et amine A), comme suit : pour B), 303,45 g de triméthylol propane (4 éthoxy) triacrylate (TMP4EOTA) (0,6429 mole) et pour A), 36,42 g de diméthylamino propylamine (DMAPA) (0,3571 mole). On obtient un produit ayant un aspect limpide, jaune clair.

Exemple B2

Réalisé à l'identique par rapport à l'exemple A1, sauf qu'on modifie les réactifs acrylate B) et amine A), comme suit : pour B), 197,34 g de triméthylol propane triacrylate (TMPTA) (0,6667 mole) et pour A), 34,00 g de diméthylamino propylamine (DMAPA) (0,3333 mole). On obtient un produit ayant un aspect limpide, jaune clair.

Le tableau ci-dessous regroupe les compositions molaires des réactants A) et B) et les caractéristiques des oligomères B1 et B2 comme décrits ci-haut.

Tableau 3 : Composition molaire des réactants A) et B) et caractéristiques des oligomères B1 et B2

| Référence oligomère | B1 | B2 |
|---|---|---|
| Composant réactif (moles) | | |
| B) : TMP4EOTA | 0,6429 | |
| B) : TMPTA | | 0,6667 |
| A) : DMAPA | 0,3571 | 0,3333 |
| Caractéristiques | | |
| r | 2,70 | 3,00 |
| $t_A$ (mEq/g) | 2,32 | 2,88 |
| Viscosité à 25°C (mPa.s) | 2000 | 6400 |
| $t_{acr}$ (mEq/g) | 3,57 | 5,76 |
| $f_o$ (mEq/mole) | 4,25 | 4,00 |
| $n_{moy}$ | 1,25 | 1,00 |
| $M_{n\ calc}$ | 1190 | 694 |

Exemple C1

Réalisé à l'identique par rapport à l'exemple A1, sauf qu'on modifie les réactifs acrylate B) et amine A), comme suit : pour B), 193,71 g d'hexanediol diacrylate (HDDA) (0,8571 mole) et pour A), 28,58 g de 1,4 bis (3-aminopropyl) pipérazine (1,4BAPP) (0,1429 mole). On obtient un produit ayant un aspect limpide, jaune clair.

Exemple C2

Réalisé à l'identique par rapport à l'exemple A1, sauf qu'on modifie les réactifs acrylate B) et amine A), comme

suit : pour B), 193,71 g d'hexanediol diacrylate (HDDA) (0,8571 mole) et pour A), 20,29 g de 1,3 bis(aminométhyl) cyclohexane (1,3BAC) (0,1429 mole). On obtient un produit ayant un aspect limpide, jaune clair.

La composition molaire des réactants A) et B) et caractéristiques des oligomères C1 et C2 sont présentées au tableau 4 ci-dessous.

Tableau 4 : Composition molaire des réactants A) et B) et caractéristiques des oligomères C1 et C2

| Référence d'oligomère | C1 | C2 |
|---|---|---|
| Composant réactif (moles) | | |
| B) : HDDA | 0,8571 | 0,8571 |
| A) : 1,3BAC | | 0,1429 |
| A) : 1,4BAPP | 0,1429 | |
| Caractéristiques | | |
| r | 3,00 | 3,00 |
| $t_A$ (mEq/g) | 2,57 | 1,34 |
| Viscosité à 25°C (mPa.s) | 955 | 340 |
| $t_{acr}$ (mEq/g) | 5,14 | 5,34 |
| $f_o$ (mEq/mole) | 2,67 | 2,67 |
| $n_{moy}$ | 0,33 | 0,33 |
| $M_{n\ calc}$ | 519 | 499 |

Exemple D1

Réalisé à l'identique par rapport à l'exemple A1, sauf qu'on modifie les réactifs acrylate B) et amine A), comme suit : pour B), 380,45 g de triméthylol propane (3 éthoxy) triacrylate (TMP3EOTA) (0,8889 mole) et pour A), 22,22 g de 1,4 bis (3-aminopropyl) pipérazine (1,4BAPP) (0,1111 mole). On obtient un produit ayant un aspect limpide, jaune clair.

Exemple D2

Réalisé à l'identique par rapport à l'exemple A1, sauf qu'on modifie les réactifs acrylate B) et amine A), comme suit : pour B), 380,49 g de de triméthylol propane (3 éthoxy) triacrylate (TMP3EOTA) (0,8889 mole) et pour A), 15,78 g de 1,3 bis(aminométhyl) cyclohexane (1,3BAC) (0,1111 mole). On obtient un produit ayant un aspect limpide, jaune clair.

La composition molaire des réactants A) et B) et caractéristiques des oligomères D1 et D2 sont présentées au tableau 5 ci-dessous.

Tableau 5 : Composition molaire et caractéristiques des oligomères D1 et D2

| Référence oligomère | D1 | D2 |
|---|---|---|
| Composant réactif (moles) | | |
| TMP3EOTA | 0,8889 | 0,8889 |
| 1,3BAC | | 0,1111 |
| 1,4BAPP | 0,1111 | |
| Caractéristiques | | |
| r | 6,00 | 6,00 |
| $t_A$ (mEq/g) | 1,10 | 0,56 |
| Viscosité à 25°C (mPa.s) | 1740 | 1278 |
| $t_{acr}$ (mEq/g) | 5,52 | 5,61 |
| $f_o$ (mEq/mole) | 4,00 | 4,00 |

(suite)

| Caractéristiques | | |
|---|---|---|
| $n_{moy}$ | 0,20 | 0,20 |
| $M_{n\ calc}$ | 725 | 713 |

Les oligomères préparés selon les exemples décrits ci-haut ont été testés dans des formulations réticulables (compositions d'application) comme décrit ci-dessous.

**Formulations et préparation**

Les oligomères sont formulés par mélange à température ambiante selon les compositions suivantes F1 à F18 avec deux types de formulations testées.

**Formules impaires** (F1, F3, F5, ...F17)

Oligomère acrylate (A1 à D2) : 96% en poids
Darocure® 1173 : 4% en poids

**Formules paires** (F2, F4, F6, ...F18)

Oligomère acrylate (A1 à D2) : 96% en poids
Darocure® 1173 : 2% en poids
Benzophénone : 2% en poids

Les compositions des formulations réalisées F1 à F18 et leurs performances sont présentées aux tableaux suivants 6 à 9. Les méthodes de caractérisation desdits oligomères et de détermination des performances desdites formulations F1 à F18 et les conditions utilisées sont rappelées ci-dessous après ces tableaux 6 à 9.

Tableau 6 : Formulations F1 à F6 à base d'oligomères A1 à A3

| Référence formulation | F1 | F2 | F3 | F4 | F5 | F6 |
|---|---|---|---|---|---|---|
| Composants | | | | | | |
| A1 | 96,0 | 96,0 | | | | |
| A2 | | | 96,0 | 96,0 | | |
| A3 | | | | | 96,0 | 96,0 |
| Darocure® 1173 | 4,0 | 2,0 | 4,0 | 2,0 | 4,0 | 2,0 |
| Benzophénone | | 2,0 | | 2,0 | | 2,0 |
| Performances | | | | | | |
| Vitesse de réticulation (mm/min) | 25 | 55 | 20 | 55 | 25 | 60 |
| Dureté Persoz | 91 | 139 | 84 | 114 | 59 | 75 |
| Dureté crayon (5B - 5H) | H | H | HB | HB | B | HB |
| Flexibilité (mm) | 3 | 3 | 3 | 3 | 4 | 3 |
| Résistance solvent (s) | 300 | 300 | 300 | 300 | 54 | 126 |

Tableau 7 : Formulations F7 à F10 à base d'oligomères B1 et B2

| Référence formulation | F7 | F8 | F9 | F10 |
|---|---|---|---|---|
| Composants | | | | |
| B1 | 96,0 | 96,0 | | |
| B2 | | | 96,0 | 96,0 |
| Darocure® 1173 | 4,0 | 2,0 | 4,0 | 2,0 |
| Benzophénone | | 2,0 | | 2,0 |
| Performances | | | | |
| Vitesse de réticulation (mm/min) | 40 | 65 | 100 | |
| Dureté Persoz | 96 | 157 | 97 | |
| Dureté crayon (5B - 5H) | HB | HB | 4B | |
| Flexibilité (mm) | 4 | 4 | 20 | |
| Résistance solvent (s) | 300 | 300 | 300 | |

Tableau 8 : Formulations F11 à F14 à base d'oligomères C1 et C2

| Référence formulation | F11 | F12 | F13 | F14 |
|---|---|---|---|---|
| Composants | | | | |
| C1 | 96,0 | 96,0 | | |
| C2 | | | 96,0 | 96,0 |
| Darocure® 1173 | 4,0 | 2,0 | 4,0 | 2,0 |
| Benzophénone | | 2,0 | | 2,0 |
| Composants | | | | |
| Performances | | | | |
| Vitesse de réticulation (mm/min) | 35 | 65 | 30 | 45 |
| Dureté Persoz | 94 | 93 | 113 | 91 |
| Dureté crayon (5B - 5H) | 2H | H | HB | HB |
| Flexibilité (mm) | 6 | 4 | 8 | 6 |
| Résistance solvant (s) | 300 | 300 | 300 | 300 |

Tableau 9 : Formulations F15 à F18 à base d'oligomères D1 et D2

| Référence formulation | F15 | F16 | F17 | F18 |
|---|---|---|---|---|
| Composants | | | | |
| D1 | 96,0 | 96,0 | | |
| D2 | | | 96,0 | 96,0 |
| Darocure® 1173 | 4,0 | 2,0 | 4,0 | 2,0 |
| Benzophénone | | 2,0 | | 2,0 |
| Performances | | | | |
| Vitesse de réticulation (mm/min) | 35 | 55 | 25 | 40 |
| Dureté Persoz | 160 | 124 | 186 | 170 |

(suite)

| Référence formulation | F15 | F16 | F17 | F18 |
|---|---|---|---|---|
| Composants | | | | |
| Dureté crayon (5B - 5H) | 3H | B | HB | HB |
| Flexibilité (mm) | 15 | 10 | 20 | 15 |
| Résistance solvent (s) | 300 | 300 | 300 | 300 |

**Méthodes de détermination des caractéristiques des oligomères et des performances des formulations**

1) Aspect

On observe visuellement le produit à la lumière du jour, à travers un flacon en verre blanc de 60 ml et on distingue si le produit est :

- Limpide : aucune turbidité, il est comparable à l'eau.
- Voilé : ne permettant plus une vision nette à travers le flacon.
- Trouble : flacon opaque, aucune image ne peut être perçue à travers le flacon.

2) Viscosité : selon viscosité Noury

On mesure le temps de parcours, dans le liquide à caractériser, d'une bille d'acier soumise à sa gravité. La norme AFNOR XP.T51-213 précise en particulier la géométrie du récipient, le diamètre de la bille (2 mm) et le parcours de la bille (104 mm). Dans ces conditions, la viscosité dynamique est proportionnelle au temps de parcours de la bille avec : 1 seconde correspondant à 0,1 Pa.s.

3) Taux d'amine $t_A$ : Calcul selon la relation (6) ci-haut définie dans la description

4) Fonctionnalité $f_o$ : Calcul selon la relation (7) ci-haut définie dans la description

5) Taux d'acrylate $t_{acr}$ : Calcul selon la relation (8) ci-haut définie dans la description

6) Réactivité par vitesse de réticulation

La formulation F1 à F18 est appliquée en film de 12 μm sur une carte contraste (« Penoparc charts form 1B »® Leneta), puis est réticulée à l'aide d'une lampe UV, Fusion Hg à 120 W/cm. On mesure la vitesse de passage minimum nécessaire (en m/min) pour obtenir un film sec au toucher. Plus la vitesse est élevée, plus la formulation est réactive.

Pour les tests de dureté, flexibilité et de résistance à l'acétone, les films photoréticulés sont laissés en salle climatisée à 23°C pendant 24 heures après réticulation et avant les mesures.

7) Dureté Persoz

La formulation à examiner est appliquée en film de 100 μm sur une plaque en verre et réticulée par une lampe UV, Fusion Hg 120 W/cm à une vitesse de 8 m/min.

Le résultat est donné en nombre d'oscillations avant l'amortissement des oscillations (passage de 12° à 4° d'amplitude), d'un pendule au contact de la plaque de verre revêtue, selon la norme ISO 1522.

8) Flexibilité

La formulation examinée est appliquée en film de 100 μm sur une plaque en acier lisse de 25/10 mm d'épaisseur (D-46® Q-Panel), puis réticulée par une lampe UV, Fusion Hg 120 W/cm à une vitesse de 8 m/min.

On courbe la plaque revêtue sur des mandrins cylindriques, selon la norme ISO 1519. On exprime le résultat par la valeur (en mm) du rayon de courbure le plus faible qu'on peut infliger au revêtement sans qu'il ne se fissure, ni se décolle du support.

9) Résistance à l'acétone (résistance chimique)

La formulation examinée est appliquée en film de 12 μm sur une plaque en verre, puis réticulée par une lampe UV, Fusion Hg 120 W/cm à une vitesse de 8 m/min. On frotte le revêtement avec un chiffon imbibé d'acétone. Le résultat retenu correspond au temps (exprimé en seconde) au-delà duquel le film se décolle et/ou se désagrège.

10) Masse moléculaire moyenne en nombre Mn : Calculée selon la relation (4) ci-haut définie dans la description

## Revendications

1. Oligomère acrylé, **caractérisé en ce qu'**il a une fonctionnalité moyenne en nombre en acrylate $f_o$ strictement supérieure à 2 acrylates par mole, qu'il a une structure ramifiée et qu'il résulte de la réaction de polyaddition d'au moins une amine multifonctionnelle A) de fonctionnalité $f_A$ en groupements N-H d'au moins 2, de préférence de 2 à 6, avec ladite fonctionnalité en N-H signifiant une fonctionnalité moyenne en nombre s'il s'agit d'un mélange d'amines, ladite amine A) étant porteuse de fonctions amines primaires et/ou secondaires, avec en option ladite amine A) pouvant être porteuse en plus d'au moins une fonction amine tertiaire (sans N-H), sur au moins un acrylate multifonctionnel B) de fonctionnalité $f_B$ en groupements acrylates d'au moins 2, de préférence de 2 à 6, avec ladite fonctionnalité en acrylates signifiant une fonctionnalité moyenne en nombre s'il s'agit d'un mélange d'acrylates, avec une fonctionnalité moyenne en nombre par mole sur l'ensemble des composants A) et B) supérieure à 2 et avec ledit oligomère comprenant dans sa structure d'unité répétitive au moins un groupement aminoacrylate $-O_2C-CH_2-CH_2-N=$ résultant de ladite polyaddition et avec un taux d'azote $t_A$, issu de ladite amine A), supérieur ou égal à 0,35 mEq/g et un rapport initial r = acrylate / N-H, compris entre $r_{inf}$ et 1,1 $r_{sup}$ et de préférence r compris entre $r_{inf}$ et $r_{sup}$, avec les valeurs $r_{inf}$ et $r_{sup}$ étant définies selon les relations (1) et (2) suivantes :

$$r_{inf} = 0{,}90*(f_A-1)*(f_B-1) \qquad (1)$$

$$r_{sup} = 2*f_A + 2*f_B - 6 \qquad (2)$$

et avec un nombre moyen $n_{moy}$ de motifs répétitifs (unités répétitives) par oligomère défini selon la relation (3) suivante :

$$n_{moy} = 1 / [(r*f_A/f_B) + 1 - f_A] \qquad (3)$$

2. Oligomère selon la revendication 1, **caractérisé en ce que** la viscosité dudit acrylate B) mesurée selon la méthode ISO 2555 à 23°C est inférieure à 200 mPa.s sous un cisaillement de 100 $s^{-1}$.

3. Oligomère selon l'une des revendications 1 à 2, **caractérisé en ce que** leditacrylate B) est sélectionné parmi : b1) acrylates de polyols aliphatiques ou cycloaliphatiques éventuellement alcoxylés, b2) oligoéthers acrylates, b3) acrylates phénoliques alcoxylés ou b4) aminoacrylates acrylés ou leurs mélanges et de préférence selon b1) ou b4) ou leurs mélanges.

4. Oligomère selon l'une des revendications 1 à 3, **caractérisé en ce qu'**il a un taux de groupements acrylates $t_{acr}$ supérieur à 2,3 et de préférence de 3,5 à 10 mmoleslg ou milli équivalents/g.

5. Oligomère selon l'une des revendications 1 à 4, **caractérisé en ce qu'**il a une masse moléculaire moyenne en nombre Mn définie selon la relation (4) suivante, allant de 275 à 5000, de préférence de 300 à 3000 :

$$M_n = M_B + (n_{moy} * M_u) \qquad (4)$$

avec $M_u$ étant la masse molaire de l'unité répétitive définie selon la relation (5) suivante :

$$M_u = M_A + (f_{A2} + 2*f_{A1} - 1)*M_B \quad (5)$$

$n_{moy}$ étant le nombre moyen d'unités répétitives tel que défini dans la revendication 1,
$M_B$ étant la masse molaire de l'acrylate B),
$M_A$ étant la masse molaire de l'amine A),
$f_{A1}$ étant le nombre de fonctions $-NH_2$ d'amine primaire, par amine A) et
$f_{A2}$ étant le nombre de fonctions -NH- d'amine secondaire, par amine A).

**6.** Oligomère selon l'une des revendications 1 à 5, **caractérisé en ce qu'**il a un taux d'azote issu de l'amine $t_A$ exprimé en mEq/g allant de 0,4 à 5, de préférence de 0,45 à 4 mEq/g, avec $t_A$ étant défini selon la relation (6) suivante :

$$t_A = 1000*n_{moy}*(f_{A1} + f_{A2} + f_{A3}) / Mn \quad (6)$$

avec Mn étant la masse moléculaire moyenne en nombre telle que définie selon la revendication 5,
$f_{A1}$ étant le nombre de fonctions $-NH_2$ amine primaire, par amine A) et
$f_{A2}$ étant le nombre de fonctions -NH- amine secondaire, par amine A)
$f_{A3}$ étant le nombre de fonctions -N= amine tertiaire, par amine A).

**7.** Oligomère selon l'une des revendications 1 à 6, **caractérisé en ce que** ladite amine A) a une fonctionnalité $f_A$ supérieure ou égale à 2 etque leditacrylate B) a une fonctionnalité $f_B$ supérieure ou égale à 3 ou inversement ladite amine A) a une fonctionnalité $f_A$ supérieure ou égale à 3 et que ledit acrylate B) a une fonctionnalité $f_B$ supérieure ou égale à 2.

**8.** Oligomère selon l'une des revendications 1 à 7, **caractérisé en ce que** :

- ladite amine A) est de formule générale $(NH_2)_{fA1}R2(NHR3)_{fA2}$, avec $R2 = R'(NR''R''')_{fA3}$, R3, R', R'' et R''' étant des alkyles en $C_1$ à $C_3$ identiques ou différents avec $f_{A1}$ étant égal à 0 et étant le nombre de fonctions amines primaires par mole, $f_{A2}$ étant supérieur ou égal à 2 et étant le nombre de fonctions amines secondaires par mole et $f_{A3}$ étant supérieur ou égal à 0 et étant le nombre de fonctions amines tertiaires par mole et avec $f_A = f_{A2}$
- ledit acrylate B) est de formule générale $R1(X)_{fB}$ avec X étant un groupement acrylate $CH_2=CH-CO_2-$ et R1 étant le résidu dudit acrylate B) porteur de $f_B$ acrylates par mole
- ledit oligomère comprend le produit de la formule générale (I) suivante :

$$X_{(fB-1)}R1[O_2CCH_2CH_2NR3R2Y_{(fA2-2)}NR3CH_2CH_2CO_2R1X_{(fB-2)}]_nX \quad (I)$$

avec $Y = -(NR3CH_2CH_2COOR1X_{(fB-1)})$.

**9.** Oligomère selon l'une des revendications 1 à 7, **caractérisé en ce que** :

- ladite amine A) est de formule générale $(NH_2)_{fA1}R2(NHR3)_{fA2}$, avec $R2 = R'(NR''R''')_{fa3}$, R3, R', R'' et R''' étant des alkyles en $C_1$ à $C_3$ identiques ou différents avec $f_{A1}$ étant supérieur ou égal à 1 et étant le nombre de fonctions amines primaires par mole, $f_{A2}$ étant supérieur ou égal à 0 et étant le nombre de fonctions amines secondaires par mole et $f_{A3}$ étant supérieur ou égal à 0 et étant le nombre de fonctions amines tertiaires par mole et avec

$$f_A = 2*f_{A1} + f_{A2}$$

- ledit acrylate B) est de formule générale $R1(X)_{fB}$ avec X étant un groupement acrylate $CH_2=CH-CO_2-$ et R1 étant le résidu dudit acrylate B) porteur de $f_B$ acrylates par mole
- ledit oligomère comprend le produit de formule générale (II) suivante :

$$X_{(fB-1)}R1[O_2CCH_2CH_2NR2Y_{fA2}Z_{(fA1-1)}CH_2CH_2CO_2R1X_{(fB-2)}]_nX \quad (II)$$

avec Y = $-(NR3CH_2CH_2CO_2R1X_{(fB-1)})$ et
Z = $-(N(CH_2CH_2COOR1X_{(fB-1)})_2)$.

**10.** Oligomère selon la revendication 7, **caractérisé en ce que** ladite fonctionnalité $f_A$ est égale à 3 et que ladite amine A) est sélectionnée parmi une diamine porteuse d'une fonction amine primaire et d'une fonction amine secondaire ou une triamine porteuse de 3 fonctions amines secondaires.

**11.** Oligomère selon la revendication 7, **caractérisé en ce que** ladite amine A) a une fonctionnalité $f_A$ égale à 2 et est une amine primaire ou une diamine porteuse de 2 fonctions amines secondaires et que ledit acrylate B) est un triacrylate éventuellement alcoxylé, avec dans le cas où ledit triacrylate est alcoxylé un nombre de motifs alcoxy par acrylate ne dépassant pas 3 si ledit alcoxy est l'éthoxy et un nombre de motifs alcoxy par acrylate ne dépassant pas 1 si ledit alcoxy est propoxy.

**12.** Oligomère selon la revendication 7, **caractérisé en ce que** ladite amine A) a une fonctionnalité $f_A$ égale à 3, qui est une diamine primaire-secondaire ou une triamine porteuse de 3 fonctions amines secondaires et que ledit acrylate B) est un diacrylate éventuellement alcoxylé, avec dans le cas où ledit diacrylate est alcoxylé un nombre de motifs alcoxy par acrylate ne dépassant pas 2 si ledit alcoxy est l'éthoxy et un nombre de motifs alcoxy par acrylate ne dépassant pas 1 si ledit alcoxy est le propoxy.

**13.** Oligomère selon la revendication 7, **caractérisé en ce que** ladite fonctionnalité $f_A$ est égale à 4 et ladite amine A) est sélectionnée parmi une diamine porteuse de 2 fonctions amines primaires ou une triamine porteuse de 1 fonction amine primaire et de 2 fonctions amines secondaires ou une tetra-amine porteuse de 4 fonctions amines secondaires.

**14.** Oligomère selon la revendication 7, **caractérisé en ce que** ladite fonctionnalité $f_A$ est égale à 5 et ladite amine A) est sélectionnée parmi une triamine porteuse de deux fonctions amines primaires et d'une fonction amine secondaire ou d'une tetra-amine porteuse d'une fonction amine primaire et de trois fonctions amines secondaires ou une penta-amine porteuse de 5 fonctions amines secondaires.

**15.** Oligomère selon la revendication 7, **caractérisé en ce que** ladite fonctionnalité $f_A$ est égale à 6 et que ladite amine A) est sélectionnée parmi une triamine porteuse de trois fonctions amines primaires ou une tetra-amine porteuse de 2 fonctions amines primaires et de 2 fonctions amines secondaires ou d'une penta-amine porteuse de 1 fonction amine primaire et de 4 fonctions amines secondaires ou une hexa-amine porteuse de 6 fonctions amines secondaires.

**16.** Oligomère selon l'une des revendications 1 à 15, **caractérisé en ce qu'**il résulte de la réaction entre au moins deux amines A) et/ou d'au moins deux acrylates B).

**17.** Oligomère selon l'une des revendications 1 à 16, **caractérisé en ce qu'**il comprend n unités répétitives avec une distribution moléculaire en fonction de n comprenant au moins les 4 produits correspondant à : n = 0 et n = 1 et n = 2 et n = 3 et plus particulièrement avec au moins 50% en poids de ladite distribution correspondant à n inférieur ou égal à 3.

**18.** Procédé de préparation d'un oligomère tel que défini selon l'une des revendications 1 à 17, **caractérisé en ce qu'**il comprend une étape de réaction de polyaddition (de Michael) de ladite au moins une amine A) sur ledit au moins un acrylate B), en présence d'un inhibiteur de polymérisation radicalaire et en l'absence de tout solvant, de tout catalyseur et de tout autre réactant hormis ladite amine A) et ledit acrylate B) et inhibiteur, ledit procédé comprenant l'addition progressive et continue de ladite amine A) sur ledit acrylate B) déjà présent dans le réacteur et toujours en excès stoechiométrique et avec ladite réaction ayant lieu à une température supérieure à 40°C et inférieure à 90°C, de préférence de 60 à 80°C et avec ladite réaction étant arrêtée à un taux de conversion des fonctions amines N-H à au moins 95% et avec un rapport initial r de fonctions acrylates sur fonctions amines N-H, r = acrylate / N-H étant entre $r_{inf}$ et 1,1 $r_{sup}$ et de préférence r compris entre $r_{inf}$ et $r_{sup}$, avec les valeurs $r_{inf}$ et $r_{sup}$ étant définies selon les relations (1) et (2) suivantes :

$$r_{inf} = 0,90*(f_A-1)*(f_B -1) \qquad (1)$$

$$r_{sup} = 2*f_A + 2*f_B - 6 \qquad (2)$$

**19.** Composition réticulable, **caractérisée en ce qu'**elle comprend au moins un oligomère tel que défini selon l'une des revendications 1 à 17 ou obtenu par le procédé tel que défini selon la revendication 18, avec en option présence d'un diluant réactif, en particulier choisi parmi les monomères (méth)acryliques mono et/ou multifonctionnels et pouvant être identique audit acrylate de départ B).

**20.** Composition selon la revendication 19, **caractérisée en ce qu'**il s'agit d'une composition réticulable par rayonnement, de préférence parmi UV, laser, LED ou EB, qui est une composition de revêtements, en particulier d'encres, de vernis, d'enduits gélifiés, de peintures ou d'adhésifs, en particulier d'adhésifs structuraux ou est une composition pour articles en 3D par superposition de couches successives ou est une composition de moulage.

**21.** Utilisation d'un oligomère, tel que défini selon l'une des revendications 1 à 17, comme liant dans des compositions réticulables.

**22.** Utilisation selon la revendication 21, **caractérisée en ce que** ladite composition est réticulable par rayonnement, de préférence parmi UV, laser, LED ou EB, ladite composition étant une composition de revêtements, en particulier d'encres, de vernis, d'enduits gélifiés, de peintures ou d'adhésifs, en particulier d'adhésifs structuraux ou étant une composition pour articles en 3D par superposition de couches successives ou étant une composition de moulage.

**23.** Produit final réticulé, **caractérisé en ce qu'**il résulte de l'utilisation d'au moins un oligomère tel que défini selon l'une des revendications 1 à 17 ou obtenu par le procédé tel que défini selon la revendication 18 ou qu'il résulte de la réticulation d'une composition telle que définie selon l'une des revendications 19 ou 20 et en particulier **en ce qu**'il s'agit de film de revêtement, plus particulièrement de film d'encre, de vernis, de revêtement d'enduits gélifiés, d'adhésif ou d'un article en 3D ou d'un joint de scellage chimique ou d'une pièce moulée.

## Patentansprüche

**1.** Acryliertes Oligomer, **dadurch gekennzeichnet, dass** es eine zahlenmittlere Acrylatfunktionalität $f_o$ von strikt höher als 2 Acrylate pro Mol aufweist, dass es eine verzweigte Struktur besitzt und dass es aus der Polyadditionsreaktion mindestens eines multifunktionellen Amins A) mit einer N-H-Gruppen-Funktionalität $f_A$ von mindestens 2, vorzugsweise von 2 bis 6, wobei die N-H-Funktionalität eine zahlenmittlere Funktionalität bedeutet, wenn es sich um ein Gemisch von Aminen handelt, wobei das Amin A) primäre und/oder sekundäre Aminfunktionen trägt, wobei optional das Amin A) zusätzlich mindestens eine tertiäre Aminfunktion (ohne N-H) tragen kann, an mindestens ein multifunktionales Acrylat B) mit einer Acrylatgruppen-Funktionalität $f_B$ von mindestens 2, vorzugsweise von 2 bis 6, wobei die Acrylat-Funktionalität eine zahlenmittlere Funktionalität bedeutet, wenn es sich um ein Gemisch von Acrylaten handelt, resultiert, wobei die zahlenmittlere Funktionalität pro Mol an den gesamten Bestandteilen A) und B) mehr als 2 beträgt, und wobei das Oligomer in seiner Wiederholungseinheitenstruktur mindestens eine aus der Polyaddition resultierte Aminoacrylatgruppe $-O_2C-CH_2-CH_2-N=$ umfasst, und wobei ein von dem Amin A) stammender Stickstoffanteil $t_A$ höher als oder gleich 0,35 mÄq/g ist und ein Anfangsverhältnis r = Acrylat/N-H zwischen $r_{inf}$ und 1,1 $r_{sup}$ liegt und vorzugsweise r zwischen $r_{inf}$ und $r_{sup}$ liegt, wobei die Werte $r_{inf}$ und $r_{sup}$ gemäß den folgenden Gleichungen (1) und (2) definiert sind:

$$r_{inf} = 0,90*(f_A - 1)*(f_B - 1) \qquad (1)$$

$$r_{sup} = 2*f_A + 2*f_B - 6 \qquad (2),$$

und wobei eine mittlere Anzahl $n_{moy}$ an Wiederholungsmotiven (Wiederholungseinheiten) pro Oligomer gemäß der folgenden Gleichung (3) definiert ist:

$$n_{moy} = 1/[(r*f_A/f_B) + 1 - f_A] \qquad (3).$$

2. Oligomer nach Anspruch 1, **dadurch gekennzeichnet, dass** die gemäß der Methode ISO 2555 bei 23°C gemessene Viskosität des Acrylats B) kleiner als 200 mPa.s unter einer Scherkraft von 100 $s^{-1}$ ist.

3. Oligomer nach einem der Ansprüche 1 bis 2, **dadurch gekennzeichnet, dass** das Acrylat B) ausgewählt ist aus: b1) gegebenenfalls alkoxylierten, aliphatischen oder cycloaliphatischen Polyolacrylaten, b2) Oligoetheracrylaten, b3) alkoxylierten Phenolacrylaten oder b4) acrylierten Aminoacrylaten oder deren Gemischen und vorzugsweise gemäß b1) oder b4) oder deren Gemischen.

4. Oligomer nach einem der Ansprüche 1 bis 3, **dadurch gekennzeichnet, dass** es einen Anteil an Acrylatgruppen $t_{acr}$ von mehr als 2,3 und vorzugsweise von 3,5 bis 10 mmol/g oder Milliäquivalenten/g aufweist.

5. Oligomer nach einem der Ansprüche 1 bis 4, **dadurch gekennzeichnet, dass** es eine gemäß der folgenden Gleichung (4) definierte zahlenmittlere Molekülmasse Mn von 275 bis 5000, vorzugsweise von 300 bis 3000, aufweist:

$$M_n = M_B + (n_{moy} * M_u) \qquad (4),$$

wobei $M_u$ die gemäß der folgenden Gleichung (5) definierte Molmasse der Wiederholungseinheit ist:

$$M_u = M_A + (f_{A2} + 2*f_{A1} - 1)*M_B \qquad (5),$$

wobei $n_{moy}$ die mittlere Anzahl an Wiederholungseinheiten, wie in Anspruch 1 definiert, ist,

$M_B$ die Molmasse des Acrylats B) ist,
$M_A$ die Molmasse des Amins A) ist,
$f_{A1}$ die Anzahl an primären Aminfunktionen $-NH_2$ pro Amin A) ist und
$f_{A2}$ die Anzahl an sekundären Aminfunktionen -NH- pro Amin A) ist.

6. Oligomer nach einem der Ansprüche 1 bis 5, **dadurch gekennzeichnet, dass** es einen aus dem Amin stammenden Stickstoffanteil $t_A$, ausgedrückt in mÄq/g, von 0,4 bis 5, vorzugsweise von 0,45 bis 4 mÄq/g, aufweist, wobei $t_A$ gemäß der folgenden Gleichung (6) definiert ist:

$$t_A = 1000*n_{moy}*(f_{A1} + f_{A2} + f_{A3})/Mn \qquad (6),$$

wobei Mn die zahlenmittlere Molekülmasse nach Anspruch 5 ist,
$f_{A1}$ die Anzahl an primären Aminfunktionen $-NH_2$ pro Amin A) ist und
$f_{A2}$ die Anzahl an sekundären Aminfunktionen -NH- pro Amin A) ist,
$f_{A3}$ die Anzahl an tertiären Aminfunktionen -N= pro Amin A) ist.

7. Oligomer nach einem der Ansprüche 1 bis 6, **dadurch gekennzeichnet, dass** das Amin A) eine Funktionalität $f_A$ von mehr als oder gleich 2 hat und dass das Acrylat B) eine Funktionalität $f_B$ von mehr als oder gleich 3 hat oder umgekehrt das Amin A) eine Funktionalität $f_A$ von mehr als oder gleich 3 hat und dass das Acrylat B) eine Funktionalität $f_B$ von mehr als oder gleich 2 hat.

8. Oligomer nach einem der Ansprüche 1 bis 7, **dadurch gekennzeichnet, dass**:

- das Amin A) die allgemeine Formel $(NH_2)_{fA1}R2(NHR3)_{fA2}$ mit R2 = $R'(NR''R''')_{fA3}$ hat, wobei R3, R', R" und R‴ gleiche oder verschiedene $C_1$- bis $C_3$-Alkyle sind, wobei $f_{A1}$ gleich 0 ist und die Anzahl an primären Aminfunktionen pro Mol ist, $f_{A2}$ größer als oder gleich 2 ist und die Anzahl an sekundären Aminfunktionen pro Mol ist und $f_{A3}$ größer als oder gleich 0 ist und die Anzahl an tertiären Aminfunktionen pro Mol ist und wobei $f_A = f_{A2}$
- das Acrylat B) die allgemeine Formel $R1(X)_{fB}$ hat, wobei X eine Acrylatgruppe $CH_2=CH-CO_2-$ ist und R1 der Rest des Acrylats B) ist, der $f_B$ Acrylate pro Mol trägt
- das Oligomer das Produkt der folgenden allgemeinen Formel (I) umfasst:

$$X_{(fB-1)}R1[O_2CCH_2CH_2NR3R2Y_{(fA2-2)}NR3CH_2CH_2CO_2R1X_{(fB-2)}]_nX \qquad (I),$$

wobei Y = - ($NR3CH_2CH_2COOR1X_{(fB-1)}$).

9. Oligomer nach einem der Ansprüche 1 bis 7, **dadurch gekennzeichnet, dass**:

- das Amin A) die allgemeine Formel $(NH_2)_{fA1}R2(NHR3)_{fA2}$ mit R2 = $R'(NR''R''')_{fA3}$ hat, wobei R3, R', R" und R‴ gleiche oder verschiedene $C_1$- bis $C_3$-Alkyle sind, wobei $f_{A1}$ größer als oder gleich 1 ist und die Anzahl an primären Aminfunktionen pro Mol ist, $f_{A2}$ größer als oder gleich 0 ist und die Anzahl an sekundären Aminfunktionen pro Mol ist und $f_{A3}$ größer als oder gleich 0 ist und die Anzahl an tertiären Aminfunktionen pro Mol ist und wobei

$$f_A = 2*f_{A1} + f_{A2}$$

- das Acrylat B) die allgemeine Formel $R1(X)_{fB}$ hat, wobei X eine Acrylatgruppe $CH_2$=CH-$CO_2$- ist und R1 der Rest des Acrylats B) ist, der $f_B$ Acrylate pro Mol trägt
- das Oligomer das Produkt der folgenden allgemeinen Formel (II) umfasst:

$$X_{(fB-1)}R1[O_2CCH_2CH_2NR2Y_{fA2}Z_{(fA1-1)}CH_2CH_2CO_2R1X_{(fB-2)}]_nX \qquad (II),$$

wobei Y = - ($NR3CH_2CH_2CO_2R1X_{(fB-1)}$) und
Z = - ($N(CH_2CH_2COOR1X_{(fB-1)})_2$).

10. Oligomer nach Anspruch 7, **dadurch gekennzeichnet, dass** die Funktionalität $f_A$ gleich 3 ist und dass das Amin A) ausgewählt ist aus einem Diamin, das eine primäre Aminfunktion und eine sekundäre Aminfunktion trägt, oder einem Triamin, das 3 sekundäre Aminfunktionen trägt.

11. Oligomer nach Anspruch 7, **dadurch gekennzeichnet, dass** das Amin A) eine Funktionalität $f_A$ gleich 2 aufweist und ein primäres Amin oder ein Diamin, das 2 sekundäre Aminfunktionen trägt, ist, und dass das Acrylat B) ein gegebenenfalls alkoxyliertes Triacrylat ist, wobei in dem Fall, wenn das Triacrylat alkoxyliert ist, eine Anzahl an Alkoxymotiven pro Acrylat 3 nicht überschreitet, wenn das Alkoxy Ethoxy ist, und eine Anzahl an Alkoxymotiven pro Acrylat 1 nicht überschreitet, wenn das Alkoxy Propoxy ist.

12. Oligomer nach Anspruch 7, **dadurch gekennzeichnet, dass** das Amin A) eine Funktionalität $f_A$ gleich 3 aufweist, wobei es sich um ein primäres-sekundäres Diamin oder ein Triamin, das 3 sekundäre Aminfunktionen trägt, handelt, und dass das Acrylat B) ein gegebenenfalls alkoxyliertes Diacrylat ist, wobei in dem Fall, wenn das Diacrylat alkoxyliert ist, eine Anzahl an Alkoxymotiven pro Acrylat 2 nicht überschreitet, wenn das Alkoxy Ethoxy ist, und eine Anzahl an Alkoxymotiven pro Acrylat 1 nicht überschreitet, wenn das Alkoxy Propoxy ist.

13. Oligomer nach Anspruch 7, **dadurch gekennzeichnet, dass** die Funktionalität $f_A$ gleich 4 ist und das Amin A) ausgewählt ist aus einem Diamin, das 2 primäre Aminfunktionen trägt, oder einem Triamin, das 1 primäre Aminfunktion und 2 sekundäre Aminfunktionen trägt, oder einem Tetraamin, das 4 sekundäre Aminfunktionen trägt.

14. Oligomer nach Anspruch 7, **dadurch gekennzeichnet, dass** die Funktionalität $f_A$ gleich 5 ist und das Amin A) ausgewählt ist aus einem Triamin, das zwei primäre Aminfunktionen und eine sekundäre Aminfunktion trägt, oder einem Tetraamin, das eine primäre Aminfunktion und drei sekundäre Aminfunktionen trägt, oder einem Pentaamin, das 5 sekundäre Aminfunktionen trägt.

15. Oligomer nach Anspruch 7, **dadurch gekennzeichnet, dass** die Funktionalität $f_A$ gleich 6 ist und das Amin A) ausgewählt ist aus einem Triamin, das drei primäre Aminfunktionen trägt, oder einem Tetraamin, das 2 primäre Aminfunktionen und 2 sekundäre Aminfunktionen trägt, oder einem Pentaamin, das 1 primäre Aminfunktion und 4 sekundäre Aminfunktionen trägt, oder einem Hexaamin, das 6 sekundäre Aminfunktionen trägt.

16. Oligomer nach einem der Ansprüche 1 bis 15, **dadurch gekennzeichnet, dass** es aus der Reaktion zwischen mindestens zwei Aminen A) und/oder mindestens zwei Acrylaten B) resultiert.

17. Oligomer nach einem der Ansprüche 1 bis 16, **dadurch gekennzeichnet, dass** es n Wiederholungseinheiten mit einer Molekülverteilung als Funktion von n umfasst, die mindestens die 4 Produkte umfasst, die Folgendem entsprechen: n = 0 und n = 1 und n = 2 und n = 3 und wobei insbesondere mindestens 50 Gew.-% der Verteilung n

kleiner als oder gleich 3 entsprechen.

**18.** Verfahren zur Herstellung eines Oligomers nach einem der Ansprüche 1 bis 17, **dadurch gekennzeichnet, dass** es einen (Michael-)Polyadditions-Reaktionsschritt des mindestens einen Amins A) an das mindestens eine Acrylat B) in Gegenwart eines Inhibitors der Radikalpolymerisation und in Abwesenheit jeglichen Lösungsmittels, jeglichen Katalysators und jegliches anderen Reaktanten mit Ausnahme des Amins A) und des Acrylats B) und des Inhibitors umfasst, wobei das Verfahren die progressive und kontinuierliche Addition des Amins A) an das Acrylat B), das bereits im Reaktor und immer im stöchiometrischen Überschuss vorhanden ist, umfasst, und wobei die Reaktion bei einer Temperatur über 40°C und unter 90°C, vorzugsweise von 60 bis 80°C, stattfindet und wobei die Reaktion bei einer Umwandlungsrate der Aminfunktionen N-H zu mindestens 95% angehalten wird, und wobei ein Anfangsverhältnis r der Acrylatfunktionen zu den Aminfunktionen N-H, r = Acrylat/N-H, zwischen $r_{inf}$ und 1,1 $r_{sup}$ liegt und vorzugsweise r zwischen $r_{inf}$ und $r_{sup}$ liegt, wobei die Werte $r_{inf}$ und $r_{sup}$ gemäß den folgenden Gleichungen (1) und (2) definiert sind:

$$r_{inf} = 0{,}90*(f_A - 1)*(f_B - 1) \quad (1)$$

$$r_{sup} = 2*f_A + 2*f_B - 6 \quad (2)$$

**19.** Vernetzbare Zusammensetzung, **dadurch gekennzeichnet, dass** sie mindestens ein Oligomer nach einem der Ansprüche 1 bis 17 oder erhalten durch das Verfahren nach Anspruch 18, gegebenenfalls in Anwesenheit eines Reaktivverdünners, der insbesondere aus mono- und/oder multifunktionalen (Meth)AcrylMonomeren, und mit dem Ausgangs-Acrylat B) identisch sein kann, ausgewählt ist, umfasst.

**20.** Zusammensetzung nach Anspruch 19, **dadurch gekennzeichnet, dass** es sich um eine durch Strahlung, vorzugsweise aus UV, Laser, LED oder EB, vernetzbare Zusammensetzung handelt, die eine Zusammensetzung von Überzugsmitteln, insbesondere Tinten, Lacken, Gelbeschichtungen, Farben oder Klebstoffen, insbesondere Strukturklebstoffen, ist oder eine Zusammensetzung für 3D-Gegenstände durch Übereinanderlegen aufeinanderfolgender Schichten ist oder eine Formmasse ist.

**21.** Verwendung eines Oligomers nach einem der Ansprüche 1 bis 17 als Bindemittel in vernetzbaren Zusammensetzungen.

**22.** Verwendung nach Anspruch 21, **dadurch gekennzeichnet, dass** die Zusammensetzung durch Strahlung, vorzugsweise aus UV, Laser, LED oder EB, vernetzbar ist, wobei die Zusammensetzung eine Zusammensetzung von Überzugsmitteln, insbesondere Tinten, Lacken, Gelbeschichtungen, Farben oder Klebstoffen, insbesondere Strukturklebstoffen, ist oder eine Zusammensetzung für 3D-Gegenstände durch Übereinanderlegen aufeinanderfolgender Schichten ist oder eine Formmasse ist.

**23.** Vernetztes Endprodukt, **dadurch gekennzeichnet, dass** es aus der Verwendung mindestens eines Oligomers nach einem der Ansprüche 1 bis 17 oder erhalten durch das Verfahren nach Anspruch 18 resultiert oder dass es aus der Vernetzung einer Zusammensetzung nach einem der Ansprüche 19 oder 20 resultiert, und insbesondere dadurch, dass es sich um einen Überzugsfilm, insbesondere einen Tinten-, Lack-, Gelbeschichtungsüberzugs-, Klebstofffilm, oder einen 3D-Artikel oder eine chemische Versiegelung oder ein Formteil handelt.

## Claims

**1.** Acrylated oligomer, **characterized in that** it has a number-average acrylate functionality $f_o$ of strictly greater than 2 acrylates per mole, that it has a branched structure and that it results from the polyaddition reaction of at least one multifunctional amine A) having a functionality $f_A$ of N-H groups of at least 2, preferably of 2 to 6, with said N-H functionality meaning a number-average functionality if it is a mixture of amines, said amine A) bearing primary and/or secondary amine functions, with optionally said amine A) possibly bearing in addition at least one tertiary amine function (without N-H), to at least one multifunctional acrylate B) having a functionality $f_B$ of acrylate groups of at least 2, preferably of 2 to 6, with said acrylate functionality meaning a number-average functionality if it is a mixture of acrylates, with a number-average functionality per mole of all of the components A) and B) of greater than 2 and with said oligomer comprising in its repeating unit structure at least one $-O_2C-CH_2-CH_2-N=$ aminoacrylate

group resulting from said polyaddition and with a content of nitrogen $t_A$, resulting from said amine A), of greater than or equal to 0.35 mEq/g and an initial ratio r = acrylate/N-H of between $r_{inf}$ and 1.1 $r_{sup}$ and preferably r between $r_{inf}$ and $r_{sup}$ with the values $r_{inf}$ and $r_{sup}$ being defined according to equations (1) and (2) below:

$$r_{inf} = 0.90*(f_A - 1)*(f_B - 1) \qquad (1)$$

$$r_{sup} = 2*f_A + 2*f_B - 6 \qquad (2)$$

and with an average number $n_{av}$ of repeating units per oligomer defined according to equation (3) below:

$$n_{av} = 1 / [(r*f_A/f_B) + 1 - f_A] \qquad (3).$$

2. Oligomer according to Claim 1, **characterized in that** the viscosity of said acrylate B) measured according to the ISO 2555 method at 23°C is less than 200 mPa.s under a shear of 100 $s^{-1}$.

3. Oligomer according to either of Claims 1 and 2, **characterized in that** said acrylate B) is selected from: b1) optionally alkoxylated aliphatic or cycloaliphatic polyol acrylates, b2) oligoether acrylates, b3) alkoxylated phenolic acrylates or b4) acrylated aminoacrylates or mixtures thereof and preferably from b1) or b4) or mixtures thereof.

4. Oligomer according to one of Claims 1 to 3, **characterized in that** it has a content of acrylate groups $t_{acr}$ of greater than 2.3 and preferably of 3.5 to 10 mmol/g or milliequivalents/g.

5. Oligomer according to one of Claims 1 to 4, **characterized in that** it has a number-average molecular mass Mn defined according to equation (4) below, ranging from 275 to 5000 and preferably from 300 to 3000:

$$M_n = M_B + (n_{av} * M_u) \qquad (4)$$

with $M_u$ being the molar mass of the repeating unit defined according to equation (5) below:

$$M_u = M_A + (f_{A2} + 2*f_{A1} - 1)*M_B \qquad (5)$$

$n_{av}$ being the average number of repeating units as defined in Claim 1,
$M_B$ being the molar mass of the acrylate B),
$M_A$ being the molar mass of the amine A),
$f_{A1}$ being the number of primary amine $-NH_2$ functions, per amine A) and
$f_{A2}$ being the number of secondary amine -NH- functions, per amine A).

6. Oligomer according to one of Claims 1 to 5, **characterized in that** it has a content of nitrogen resulting from the amine $t_A$ expressed in mEq/g ranging from 0.4 to 5, preferably from 0.45 to 4 mEq/g, with $t_A$ being defined according to equation (6) below:

$$t_A = 1000*n_{av} *(f_{A1} + f_{A2} + f_{A3}) / Mn \qquad (6)$$

with Mn being the number-average molecular mass as defined according to Claim 5,
$f_{A1}$ being the number of primary amine $-NH_2$ functions, per amine A) and
$f_{A2}$ being the number of secondary amine -NH- functions, per amine A)
$f_{A3}$ being the number of tertiary amine -N= functions, per amine A) .

7. Oligomer according to one of Claims 1 to 6, **characterized in that** said amine A) has a functionality $f_A$ greater than or equal to 2 and that said acrylate B) has a functionality $f_B$ greater than or equal to 3 or conversely said amine A) has a functionality $f_A$ greater than or equal to 3 and that said acrylate B) has a functionality $f_B$ greater than or equal to 2.

8. Oligomer according to one of Claims 1 to 7, **characterized in that**:

- said amine A) is of general formula $(NH_2)_{fA1}R2(NHR3)_{fA2}$, with R2 = R'(NR"R‴)$_{fA3}$, R3, R', R" and R‴ being identical or different $C_1$ to $C_3$ alkyls with $f_{A1}$ being equal to 0 and being the number of primary amine functions per mole, $f_{A2}$ being greater than or equal to 2 and being the number of secondary amine functions per mole and $f_{A3}$ being greater than or equal to 0 and being the number of tertiary amine functions per mole and with $f_A = f_{A2}$
- said acrylate B) is of general formula $R1(X)_{fB}$ with X being a $CH_2$=CH-$CO_2$- acrylate group and R1 being the residue of said acrylate B) bearing $f_B$ acrylates per mole
- said oligomer comprises the product of general formula (I) below:

$$X_{(fB-1)}R1[O_2CCH_2CH_2NR3R2Y_{(fA2-2)}NR3CH_2CH_2CO_2R1X_{(fB-2)}]_nX \quad (I)$$

with Y = - $(NR3CH_2CH_2COOR1X_{(fB-1)})$.

9. Oligomer according to one of Claims 1 to 7, **characterized in that**:

- said amine A) is of general formula $(NH_2)_{fA1}R2(NHR3)_{fA2}$, with R2 = R'(NR"R‴)$_{fA3}$, R3, R', R" and R‴ being identical or different $C_1$ to $C_3$ alkyls with $f_{A1}$ being greater than or equal to 1 and being the number of primary amine functions per mole, $f_{A2}$ being greater than or equal to 0 and being the number of secondary amine functions per mole and $f_{A3}$ being greater than or equal to 0 and being the number of tertiary amine functions per mole and with

$$f_A = 2*f_{A1} + f_{A2}$$

- said acrylate B) is of general formula $R1(X)_{fB}$ with X being a $CH_2$=CH-$CO_2$- acrylate group and R1 being the residue of said acrylate B) bearing $f_B$ acrylates per mole
- said oligomer comprises the product of general formula (II) below:

$$X_{(fB-1)}R1[O_2CCH_2CH_2NR2Y_{fA2}Z_{(fA1-1)}CH_2CH_2CO_2R1X_{(fB-2)}]_nX \quad (II)$$

with Y = $-(NR3CH_2CH_2CO_2R1X_{(fB-1)})$ and
Z = - $(N(CH_2CH_2COOR1X_{(fB-1)})_2)$.

10. Oligomer according to Claim 7, **characterized in that** said functionality $f_A$ is equal to 3 and that said amine A) is selected from a diamine bearing a primary amine function and a secondary amine function or a triamine bearing 3 secondary amine functions.

11. Oligomer according to Claim 7, **characterized in that** said amine A) has a functionality $f_A$ equal to 2 and is a primary amine or a diamine bearing 2 secondary amine functions and that said acrylate B) is an optionally alkoxylated triacrylate with, in the case where said triacrylate is alkoxylated, a number of alkoxy units per acrylate that does not exceed 3 if said alkoxy is ethoxy and a number of alkoxy units per acrylate that does not exceed 1 if said alkoxy is propoxy.

12. Oligomer according to Claim 7, **characterized in that** said amine A) has a functionality $f_A$ equal to 3, which is a primary-secondary diamine or a triamine bearing 3 secondary amine functions and that said acrylate B) is an optionally alkoxylated diacrylate with, in the case where said diacrylate is alkoxylated, a number of alkoxy units per acrylate that does not exceed 2 if said alkoxy is ethoxy and a number of alkoxy units per acrylate that does not exceed 1 if said alkoxy is propoxy.

13. Oligomer according to Claim 7, **characterized in that** said functionality $f_A$ is equal to 4 and said amine A) is selected from a diamine bearing 2 primary amine functions or a triamine bearing 1 primary amine function and 2 secondary amine functions or a tetra-amine bearing 4 secondary amine functions.

14. Oligomer according to Claim 7, **characterized in that** said functionality $f_A$ is equal to 5 and said amine A) is selected from a triamine bearing two primary amine functions and one secondary amine function or a tetra-amine bearing one primary amine function and three secondary amine functions or a penta-amine bearing 5 secondary amine functions.

**15.** Oligomer according to Claim 7, **characterized in that** said functionality $f_A$ is equal to 6 and that said amine A) is selected from a triamine bearing three primary amine functions or a tetra-amine bearing 2 primary amine functions and 2 secondary amine functions or a penta-amine bearing 1 primary amine function and 4 secondary amine functions or a hexa-amine bearing 6 secondary amine functions.

**16.** Oligomer according to one of Claims 1 to 15, **characterized in that** it results from the reaction between at least two amines A) and/or at least two acrylates B).

**17.** Oligomer according to one of Claims 1 to 16, **characterized in that** it comprises n repeating units with a molecular distribution as a function of n comprising at least the 4 products corresponding to: n = 0 and n = 1 and n = 2 and n = 3 and more particularly with at least 50% by weight of said distribution corresponding to n less than or equal to 3.

**18.** Process for preparing an oligomer as defined according to one of Claims 1 to 17, **characterized in that** it comprises a step of (Michael) polyaddition reaction of said at least one amine A) to said at least one acrylate B), in the presence of a radical polymerization inhibitor and in the absence of any solvent, of any catalyst and of any other reactant except for said amine A) and said acrylate B) and inhibitor, said process comprising the gradual and continuous addition of said amine A) to said acrylate B) already present in the reactor and always in stoichiometric excess and with said reaction taking place at a temperature above 40°C and below 90°C, preferably from 60°C to 80°C and with said reaction being stopped at a degree of conversion of the N-H amine functions of at least 95% and with an initial ratio r of acrylate functions to N-H amine functions, r = acrylate / N-H being between $r_{inf}$ and 1.1 $r_{sup}$ and preferably r between $r_{inf}$ and $r_{sup}$, with the values $r_{inf}$ and $r_{sup}$ being defined according to equations (1) and (2) below:

$$r_{inf} = 0.90*(f_A - 1)*(f_B - 1) \qquad (1)$$

$$r_{sup} = 2*f_A + 2*f_B - 6 \qquad (2)$$

**19.** Crosslinkable composition, **characterized in that** it comprises at least one oligomer as defined according to one of Claims 1 to 17 or obtained by the process as defined according to Claim 18, with optionally the presence of a reactive diluent, in particular selected from monofunctional and/or multifunctional (meth)acrylic monomers and that may be identical to said starting acrylate B).

**20.** Composition according to Claim 19, **characterized in that** it is a radiation-crosslinkable composition, preferably that can be crosslinked by UV, laser, LED or EB radiation, said composition being a coating composition, in particular an ink, varnish, gel coat, paint or adhesive, in particular structural adhesive, composition or being a composition for 3D articles made by superposition of successive layers or being a moulding composition.

**21.** Use of an oligomer as defined according to one of Claims 1 to 17, as binder in crosslinkable compositions.

**22.** Use according to Claim 21, **characterized in that** said composition is a radiation-crosslinkable composition, preferably that can be crosslinked by UV, laser, LED or EB radiation, said composition being a coating composition, in particular an ink, varnish, gel coat, paint or adhesive, in particular structural adhesive, composition or being a composition for 3D articles made by superposition of successive layers or being a moulding composition.

**23.** Crosslinked final product, **characterized in that** it results from the use of at least one oligomer as defined according to one of Claims 1 to 17 or obtained by the process as defined according to Claim 18 or that it results from the crosslinking of a composition as defined according to either of Claims 19 and 20 and in particular in that it is a coating film, more particularly an ink, varnish, gel coat or adhesive film or a 3D article or a chemical seal or a moulded part.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6172129 B
- WO 2011131501 A
- US 5792827 A